# EUROPÄISCHE PATENTSCHRIFT

(11) **EP 0 793 978 B1**
(45) Veröffentlichungstag und Bekanntmachung des Hinweises auf die Patenterteilung: **10.12.2003**
(21) Anmeldenummer: 97250056.5
(22) Anmeldetag: 04.03.1997
(51) Int. Cl.: A61N 1/368, A61N 1/39

(54) **Therapiegerät**
Therapy device
Appareil de thérapie

(30) Priorität: 04.03.1996 DE 19609362
(43) Veröffentlichungstag der Anmeldung: 10.09.1997
(73) Patentinhaber: BIOTRONIK Mess- und Therapiegeräte GmbH & Co Ingenieurbüro Berlin, 12359 Berlin (DE)
(72) Erfinder: Thong, Tran, Lake Oswego, Oregon 97035 (US); Schaldach, Max, Prof.-Prof.-Ing., 91054 Erlangen (DE); Digby, Dennis, Lake Oswego, Oregon 97035 (US)
(74) Vertreter: Eisenführ, Speiser & Partner

(56) Entgegenhaltungen:
- WO-A-94/16768
- US-A- 5 379 776
- US-A- 5 474 552

## Beschreibung

Die Erfindung betrifft ein Therapiegerät der im Oberbegriff des Anspruchs 1 angegebenen Art.

Selbsttätig arbeitende medizinische Therapiegeräte sind - insbesondere und seit längerem als implantierbare Herzschrittmacher zur Behandlung von bradykarden und/oder tachykarden Herzrhythmusstörungen, aber auch zunehmend als automatische Defibrillatoren bzw. Kardioverter, als kombinierte Schrittmacher/Kardioverter oder als implantierte Medikamentendosierpumpen o.ä. - allgemein bekannt und im alltäglichen medizinischen Einsatz.

Bekannt sind insbesondere gattungsgemäße Geräte, die mit einem oder mehreren Fühler(n) zur Aufnahme von diagnostisch relevanten Signalen im Körper des Patienten und zugehörigen Signalaufbereitungs- und -verarbeitungseinrichtungen sowie einer Auswertungs- und Steuereinheit ausgerüstet sind, die gemäß einem im Gerät gespeicherten Algorithmus in Abhängigkeit vom Wert bzw. den Werten der aufgenommenen Größe(n) aus der Menge der programmierten Betriebsparameter bzw. Therapiegrößen jeweils einen aktuellen Parameter bzw. Parametersatz berechnet. Hierzu zählen etwa Herzschrittmacher, bei denen die Stimulationsrate in Abhängigkeit von der körperlichen Aktivität des Trägers gesteuert wird. Weiterhin sind Therapiegeräte bekannt, die zu einer automatischen Aktivierung oder - insbesondere ebenfalls vorprogrammierten - Umschaltung aus einer Betriebsart in eine andere in Abhängigkeit vom Wert einer im Körper des Patienten erfaßten Größe ausgebildet sind. Hierzu zählen die bekannten Bedarfs-Herzschrittmacher oder automatischen Defibrillatoren und die in neuerer Zeit entwickelten Kombinationsgeräte.

Bereits seit der Entwicklung und dem klinischen Einsatz sogenannter Bedarfs-(Demand-)Schrittmacher ist es bekannt, speziell Herzschrittmacher derart zu steuern, daß die spontanen Herzaktionen erfaßt und daraus der Wert der Herzrate bzw. der Zeitintervalle zwischen bestimmten Herzaktionen (etwa der RR-Intervalle zwischen aufeinanderfolgenden Ventrikelaktionen) ermittelt und mit einem vorgegebenen Sollwert verglichen werden und der Schrittmacher genau dann Stimulationsimpulse abgibt, wenn der Meßwert nicht innerhalb eines durch den Sollwert begrenzten Bereiches liegt.

Modernere Geräte dieser Art sind mikroprozessorgesteuert und bieten die Möglichkeit der individuellen, auf ein konkretes Krankheitsbild zugeschnittenen Programmierung einer von einer Mehrzahl vorinstallierter Betriebsweisen, mit der eine vorgegebene Therapie realisiert wird, und zugehöriger Betriebsparameter (im weiteren auch als Therapiegrößen bezeichnet, soweit sie therapeutisch relevant sind).

In diesem Rahmen haben auch wesentliche Weiterentwicklungen des Konzeptes des Bedarfs-Schrittmachers hin zum universellen Bedarfs-Herzrhythmuskorrekturgerät stattgefunden, von denen einige von einer fortschreitend feineren Bereichsunterteilung des Herzraten- oder RR-Intervall-Kontinuums ausgehen und in Abhängigkeit davon, in welchem der Mehrzahl vorgegebener Bereiche ein aktueller Meßwert liegt, eine von einer Mehrzahl definierter, jeweils eindeutig einem Bereich zugeordneter Therapien realisiert wird. Mit einem solchen Gerät ist ein klassischer Bedarfs-Schrittmacherbetrieb im Fall einer Bradykardie ebenso realisierbar wie konventionelle Therapien verschiedener Tachykardien (vgl. etwa US 4 181 133) oder ggfs. auch eine Defibrillationsschock-Therapie (vgl. US 4 300 567).

Ausgehend von der Erkenntnis, daß allein die Zuordnung der Herzrate zu einem der vorgegebenen Bereiche nicht immer zuverlässig die Bestimmung der angemessenen Therapie erlaubt, wurden in den Entwicklungen der letzten Jahre verstärkt auch zusätzliche Klassifizierungskriterien getestet und in den Steueralgorithmen berücksichtigt; vgl. hierzu etwa US 5,379,776 (einschließlich darin genannter Quellen).

Geräte der genannten Art werden bei der Implantation entsprechend dem Krankheitsbild und ggfs. den Lebensbedingungen (beispielsweise der durchschnittlichen körperlichen Aktivität) des Patienten programmiert, wobei auch der anzuwendende Algorithmus zur Bestimmung der Therapie bzw. Therapiegröße(n) in Abhängigkeit vom Wert der im Körper erfaßten Größe(n) festgelegt wird. Bei den in bestimmten Abständen erfolgenden Nachsorgeuntersuchungen können durch eine Umprogrammierung sowohl der Betriebsart- und -paramatersatz als auch - falls das Therapiegerät über mehrere gespeicherte Algorithmen verfügt - der anzuwendende Steueralgorithmus geändert werden

Jedoch ist in den Betriebsphasen zwischen den Untersuchungen über den aktivierten Algorithmus eindeutig festgelegt, welche Therapie bzw. Therapiegröße das Gerät in einem bestimmten Wertebereich der im (oder am) Körper erfaßten Meßgröße liefert - beispielsweise, bei welchen Grenz-Herzraten eine vorgegebene Stimulation zur Behandlung einer Bradykardie oder Tachykardie einsetzt bzw. von einem Stimulationsmodus in einen anderen umgeschaltet wird. Diese zu einem bestimmten Zeitpunkt und aufgrund eines spezifischen körperlichen Zustands des Patienten getroffene Zuordnung muß jedoch keineswegs streng gültig sein. Insbesondere kann eine vorangegangene Therapie bzw. ein (auch vordergründig erfolgloser) Therapieversuch den körperlichen Zustand des Patienten bereits latent verändert haben.

Durch eine komplexere Klassifizierungsmethodik und/oder die im wesentlichen gleichzeitige Erfassung und Auswertung der Signale mehrerer Sensoren kann zwar der aktuelle körperliche Zustand des Patienten zunehmend genauer abgebildet werden, dies ändert jedoch nichts an der nachteiligen strengen Determiniertheit der Therapie durch die Meßgröße(n).

Der Erfindung liegt daher die Aufgabe zugrunde, ein Therapiegerät der eingangs genannten Gattung so weiterzubilden, daß dieses aufgrund eines vorgegebenen Auswertungs- und Steueralgorithmus unter Berücksichtigung der Therapie-Vorgeschichte eine einerseits möglichst schonende, aber andererseits möglichst schnell wirksame Therapie zu liefern vermag, ohne daß das Gerät wesentlich komplexer und kostenaufwendiger wird.

Diese Aufgabe wird durch ein Therapiegerät mit den Merkmalen des Anspruchs 1 gelöst.

Die Erfindung schließt den Gedanken ein, ein Gerät mit Mitteln zu schaffen, die die bisherigen Lösungen eigene strenge Determiniertheit der Therapie bzw. Therapiegröße durch die Lage einer Meßgröße in einem Bereich mit eindeutig definierten Grenzen dadurch relativieren, daß Überlappungszonen zwischen den Bereichen definiert werden, in denen in Abhängigkeit von der Therapie-Vorgeschichte jeweils verschiedene Therapien bzw. Therapiegrößen realisiert werden. Eine solche Bereichs-Überlappung in Verbindung mit einer mehrdeutigen Zuordnung von Therapien bzw. Therapiegrößen erfordert insbesondere zusätzliche Speicher- und weiterentwickelte Verarbeitungsmittel, der Zusatzaufwand ist jedoch angesichts der niedrigen spezifischen Kosten für Speicher- und Verarbeitungskapazität unproblematisch.

In einer zweckmäßigen Ausführung der Ablaufsteuerung weist diese einen Zeitgeber sowie eine durch diesen getaktete, über einen ersten Eingang mit dem Sensor für die aktuell im Körper zu erfassende Werte der Meßgröße und über einen zweiten Eingang mit dem Bereichsgrenzenspeicher verbundene Meßwert-Vergleichereinheit auf. Unter Steuerung durch den Zeitgeber wird dann periodisch der aktuelle Wert der Meßgröße abgefragt und mit den gespeicherten Grenzen verglichen und ein das Vergleichsergebnis kennzeichnendes Zugriffssteuer- und Adreßsignal ausgegeben, das ein Auslesen desjenigen Wertes der Therapiesteuergröße aus dem ersten Therapiespeicher bewirkt, mit dem eine dem zutreffenden Meßbereich zugeordnete Therapie bzw. Therapiegröße gesteuert wird - oder das ein Auslesen überhaupt unterbindet. Im letzteren Fall wird der zuletzt gültige Wert der Therapiesteuergröße, d.h. die laufende Therapie beibehalten. Die Ablaufsteuerung kann auch so ausgebildet sein, daß ein Zugriffssteuersignal zum Auslesen aus dem ersten Therapiespeicher überhaupt nur nach Feststellung der Überschreitung einer Wertebereichsgrenze ausgegeben wird.

Zur Realisierung der genannten Anordnung gehört zur Ablaufsteuerung ein Meßwertspeicher mindestens für den jeweils zuletzt abgefragten Meßwert, und die Meßwert-Vergleichereinheit ist auch mit diesem verbunden und so ausgebildet, daß das ausgegebene Zugriffssteuer- und Adreßsignal ein Überschreiten einer Bereichsgrenze gegenüber der letzten Meßwerterfassung kennzeichnet. Der Meßwertspeicher kann - mit etwas höherem Aufwand - auch als Trendspeicher für eine zeitliche Folge vorangehender Meßwerte ausgeführt sein, wobei dann anstelle der Vergleichereinheit Mittel zur Ausführung einer Trendberechnung für die Meßwerte vorgesehen sind.

In einer speziellen Ausführung als implantierbarer Bedarfsschrittmacher ist das Therapiegerät mit einem Bereichsgrenzenspeicher ausgestattet, der zur Speicherung mindestens einer Grenze zwischen einem Normal- und einem Bradykardie-Ratenbereich sowie zweier Grenzen und somit einer Überlappungszone zwischen dem Normal- und einem Tachykardie-Ratenbereich vorgesehen ist, sowie einem ersten Therapiespeicher, der zur Speicherung mindestens je einer (an sich bekannten) Bradykardie- und Tachykardie-Therapie ausgebildet ist.

Bei einer weiteren speziellen Ausführung als implantierbarer Antitachykardie-Schrittmacher/Defibrillator ist der Bereichsgrenzenspeicher zur Speicherung mindestens einer Grenze zwischen einem Normal- und einem Tachykardie-Ratenbereich sowie zweier Grenzen und somit einer Überlappungszone zwischen dem Tachykardie-Ratenbereich und einem Fibrillationsbereich ausgebildet. Weiterhin kann bei einem solchen Kombinationsgerät der Bereichsgrenzenspeicher je zwei Speicherbereiche zur Speicherung je zweier Grenzen zwischen dem Normal- und dem Tachykardie-Ratenbereich sowie dem Tachykardie-Ratenbereich und dem Fibrillationsbereich umfassen, wobei dann natürlich auch die Therapie-Zuordnung in den beiden Überlappungszonen entsprechend mehrdeutig ist. Dies gilt auch für eine weiter verfeinerte Ausführung zuur differnezierten Behandlung qualitativ verschiedenartiger Tachykardien, bei der der Bereichsgrenzenspeicher Speicherbereiche für zwei, über eine zwischen zwei Grenzen liegende Überlappungszone aneinander angrenzende, Tachykardie-Ratenbereiche aufweist.

Mit einem Schrittmacher bzw. Kombinationsgerät, bei dem der Bereichsgrenzenspeicher zwei Speicherbereiche zur Speicherung zweier Grenzen und somit einer Überlappungszone zwischen dem Normal- und dem Bradykardie-Ratenbereich aufweist, lassen sich in vorteilhafter Weise zugleich bekannte Bedarfsschrittmacher-Hysteresefunktionen ausführen.

Eine Ausführungslinie der Erfindung, mit der sich besonders empfindlich und flexibel auf Änderungen des Therapiebedarfs ansprechende Geräte realisieren lassen, schließt den Gedanken ein, ein Gerät mit Mitteln zu schaffen, die die strenge Determiniertheit der Therapie bzw. Therapiegröße durch die Meßgröße(n) dadurch relativieren, daß letzterer (letzteren) im Zuge der Auswertung eine willkürliche zeitliche Schwankung aufgeprägt wird. Hierdurch ergibt sich eine mit dem Schwankungswert korrigierte Pseudo-Meßgröße, deren weitere Auswertung mit dem festgelegten Algorithmus zur Ausgabe einer variierten Therapie (Therapiegröße) führt. Diese Variation der Therapie ermöglicht eine vergleichende Erfolgskontrolle, auf deren Grundlage (ohne Änderung des Algorithmus) die Therapie optimiert werden kann.

Die Erfolgskontrolle kann insbesondere mittels eines zusätzlichen, den körperlichen Zustand des Patienten insoweit, wie das Therapiegerät zu dessen Beeinflussung eingesetzt wird, möglichst genau abbildenden Sensors erfolgen. Alternativ hierzu kann sie über den auch zur Gewinnung der primären Meßgröße genutzten Sensor ausgeführt werden - jeweils unter Zuhilfenahme von vorab in Zuordnung zueinander gespeicherten Meßwerten und Bewertungskriterien. Die erfolgreichere bzw. erfolgreichste der variierten Therapie(n) wird dann als aktuell gültige festgelegt. Dabei können - je nach der konkreten Aufgabe und Ausbildung des Therapiegerätes - verschiedene Strategien verfolgt und entsprechende technische Mittel eingesetzt werden, worauf weiter unten genauer eingegangen wird.

Für einige Ausführungsformen sind spezielle Mittel zur vergleichenden Erfolgskontrolle sogar verzichtbar, sei es, weil die Wirkung einer einmalig variierten Therapie eine (mindestens zeitweilige) selbsttätige Aussetzung jedweder Therapie zur Folge hat - man denke an eine aufgrund des schwankungsbehafteten Meßwertes der Herzrate ausgegebene, wirksame Impulsfolge zur Beendigung einer sich gerade beschleunigenden Tachykardie oder einen auf analoge Weise gesteuerten Kardioversionsimpuls bei einsetzenden Fibrillationen oder weil eine fortgesetzte leichte Schwankung der Therapiegröße aufgrund der der Meßgröße aufgeprägten Schwankung aus grundsätzlichen Erwägungen als vorteilhaft angesehen werden kann, wie unter bestimmten Umständen bei einem Medi kamentendosiergerät.

Bei einer zweckmäßigen Ausbildung mit einem einzigen Sensor zur Steuerung und zugleich zur Erfolgskontrolle ist dem Sensor ein Vergleichswertspeicher zugeordnet und eine eingangsseitig mit den Ausgängen des Sensors und des Vergleichswertspeichers verbundene Vergleichereinheit vorgesehen, in der der Sensor-Meßwert einem Vergleich mit mindestens einem gespeicherten Vergleichswert unterzogen wird und die im Ergebnis des Vergleiches ein Steuersignal abgibt, aufgrund dessen der Sensor-Meßwert entweder dem Eingang der mathematischen Verarbeitungseinheit - zur Verarbeitung mit einem Schwankungswert - oder unter Umgehung dieser direkt dem Eingang der Auswertungs- und Steuereinrichtung zugeführt wird.

Bei einem Zwei-Sensoren-Gerät hingegen sind ein erster und ein zweiter Sensor vorgesehen, wobei dem Ausgang des ersten Sensors der Schwankungswertgenerator zugeordnet ist derart, daß mit dem Schwankungswert korrigierte Meßwerte des ersten Sensors der Variation der Therapie bzw. Therapiegröße zugrundegelegt werden, während der Ausgang des zweiten Sensors mindestens mittelbar mit einem Eingang der Zeitsteuereinheit verbunden ist derart, daß die Aufprägung der Schwankungsgröße auf die Meßwerte des ersten Sensors in Abhängigkeit von den Meßwerten des zweiten Sensors gesteuert, insbesondere wahlweise unterbunden, wird.

Der oben genannte Sensor bzw. erste Sensor kann zur Erfassung einer Aktivitätsgröße oder einer eine Organfunktion des Patienten kennzeichnenden Größe und/oder zur Erfassung der Therapiegröße - etwa als intrakardiale Elektrode mit nachgeschaltetem Abfühlverstärker für elektrische Aktivität des Herzens, speziell mit zugeordneter Einrichtung zur Bestimmung der Periode der elektrischen Herzaktivität als Meßwert - ausgebildet sein. Der zweite Sensor wird bevorzugt ein zur Erfassung einer von einer Organfunktion oder der Therapiegröße abhängigen, eher den körperlichen bzw. hämodynamischen Gesamtzustand des Patienten kennzeichnenden Größe ausgebildeter Fühler, beispielsweise ein Blutdruckfühler, sein.

Die Schwankungswert-Verarbeitung der Meßgröße kann - je nach konkreter Anwendung - in zweckmäßiger Weise durch Addition/Subtraktion oder durch Multiplikation/Division erfolgen. Entsprechend ist der Schwankungswertgenerator zur Ausgabe mindestens eines Inkrement- oder Dekrementwertes und die mathematische Verarbeitungseinheit als Additionsstufe oder der Schwankungswertgenerator zur Ausgabe mindestens eines Korrekturfaktors und die mathematische Verarbeitungseinheit als Multiplikationsstufe ausgebildet. Spezieller umfaßt der Schwankungswertgenerator einen Schwankungswertspeicher für mehrere Schwankungswerte und wahlweise einen Zufallsgenerator zur Auswahl je eines der gespeicherten Schwankungswerte zur Aufprägung auf den Meßwert.

In der Ausbildung des Therapiegerätes als, insbesondere implantierbarer, Kardioverter kann zweckmäßigerweise der bzw. der erste Sensor durch eine intrakardiale Elektrode mit nachgeschaltetem Abfühlverstärker gebildet sein und eine Einrichtung zur Bestimmung der Periode der elektrischen Herzaktivität als Meßwert aufweisen. Die Auswertungs- und Steuereinrichtung ist hierbei zur Festlegung mindestens einer vorgegebenen Folge elektrischer Stimulationsimpulse und/oder eines energiereichen Einzelimpulses und die Therapieeinrichtung zur Erzeugung und Abgabe der entsprechenden elektrischen Stimulationsimpulse ausgebildet.

Eine ganz andersartige Realisierung stellt die Ausbildung als, insbesondere implantierbares, Medikamentendosiergerät dar, bei dem der bzw. der erste Sensor zur Erfassung des Pegels eines Wirkstoffes oder einer davon abhängigen Größe im Körper des Patienten, die Auswertungs- und Steuereinrichtung zur Festlegung einer Medikamentendosis pro Zeiteinheit und die Therapieeinrichtung zur Abgabe der festgelegten Dosis pro Zeiteinheit an den Körper ausgebildet sind. In einer speziellen Ausführung zur medikamentösen Behandlung kardialer Arrythmien kann die Meßwerterfassung speziell auch hier über eine intrakardiale Elektrode erfolgen, da die Herzaktionen natürlich die zu behandelnde Arrythmie ebenso reflektieren wie den Behandlungserfolg.

Vorteilhafte Weiterbildungen der Erfindung sind in den Unteransprüchen gekennzeichnet bzw. werden nachstehend zusammen mit der Beschreibung bevorzugter Ausführungen der Erfindung anhand der Figuren näher dargestellt. Es zeigen:
Figur 1 eine schematische Darstellung des in verschiedene Abschnitte gegliederten Herzratenkontinuums zur Illustration der Arbeitsweise eines kombinierten Herzschrittmachers/Defibrillators nach einer bevorzugten Ausführungsform der Erfindung,
Figur 2 ein vereinfachtes Funktions-Blockschaltbild eines kombinierten Herzschrittmachers/Defibillators gemäß einer auf Fig. 1 bezogenen Ausführungsform der Erfindung,
Figur 3 eine schematische Darstellung zum Betrieb des kombinierten Herzschrittmachers/Defibrillators nach Fig. 2,
Figur 4 ein vereinfachtes Funktions-Blockschaltbild eines Medikamentendosiergerätes für Antiarrhythmika gemäß einer weiteren Ausführungsform der Erfindung und
Figur 4a ein vereinfachtes Funktions-Blockschaltbild einer Ausführungsform der Auswertungs- und Steuereinheit des Gerätes nach Fig. 4.

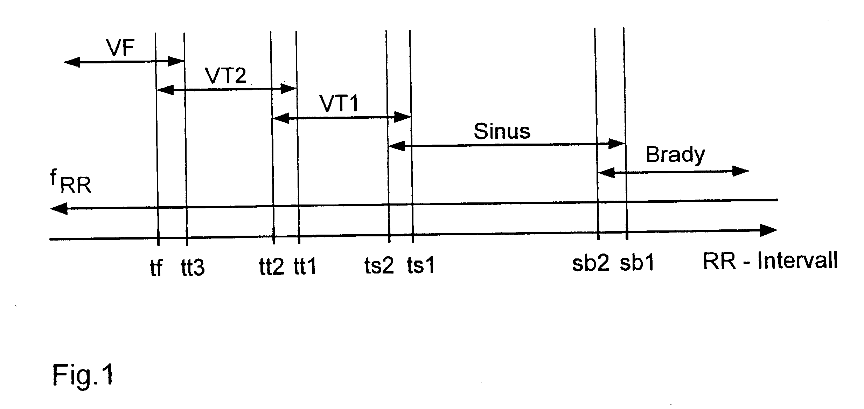

Bei der in Figur 1 wiedergegebenen schematischen Darstellung des in verschiedene, einander überlappende Abschnitte gegliederten Herzratenkontinuums zur Illustration der Arbeitsweise eines solchen Gerätes ist auf der x-Achse (mit nach rechts zunehmenden Werten) des RR-Intervall bzw. (mit nach links zunehmenden Werten) die Herzrate f_{RR} aufgetragen. Mit "VF" ist der Bereich ventrikulärere Fibrillation bezeichnet, mit "VT2" und "VT1" sind zwei aneinander angrenzende Bereiche ventrikulärer Tachykardie mit unterschiedlicher diagnostischer und therapeutischer Relevanz bezeichnet, "Sinus" bezeichnet den Bereich normaler Herztätigkeit und "Brady" den Bereich unzulässig niedriger Herzrate, d.h. einer (ventrikulären) Bradykardie. Grundsätzlich erfordert die Messung eines Wertes von f_{RR} außerhalb des Bereiches "Sinus" eine spezifische Stimulation des Herzens zur Zurückführung in den Normalbereich, wobei die Art und Weise der Stimulation und deren physiologische Auswirkungen auf den Patienten sich erheblich unterscheiden.

Mit "tf", "tt3", "tt2", "tt1", "ts2", "ts1", "sb2" und "sb1" sind die (wegen der erwähnten Überlappung jeweils doppelten) Grenzen zwischen den einzelnen Bereichen bezeichnet; zur Bedeutung der Überlappungsregionen siehe weiter unten.

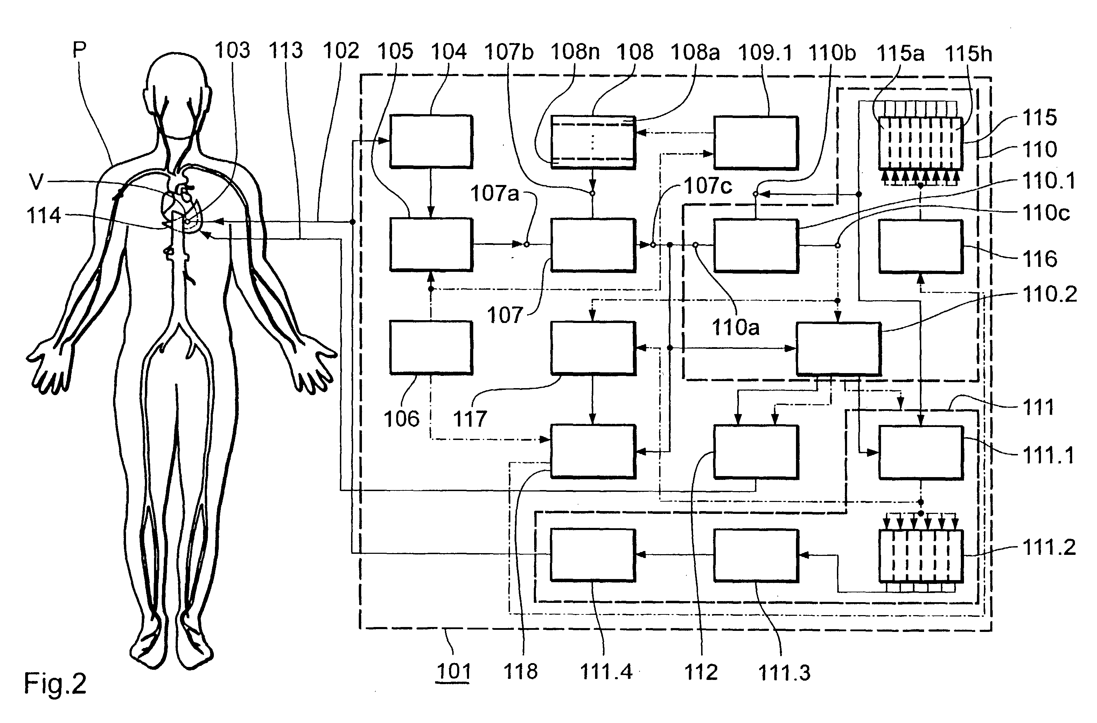

In dem vereinfachten Blockschaltbild eines kombinierten Herzschrittmachers/Defibrillators 101 gemäß Figur 2 ist - in an sich bekannter Weise - über eine Elektrodenleitung 102 mit einer ventrikulär fixierten Herzelektrode 103 verbunden.

Das proximale Ende der Elektrodenleitung 102 ist mit einer Eingangsverstärkerstufe 104 verbunden, die (in an sich von Herzschrittmachern bekanntem Aufbau) Filter- und Verstärkerstufen zur Signalaufbereitung aufweist und an deren Ausgang ein weitgehend störbefreites, pegelangepaßtes Herzsignal anliegt. Der Ausgang der Eingangsstufe 104 ist mit dem Signaleingang einer Zählerstufe 105 verbunden, deren Takteingang mit einem Takt- bzw. Zeitgeber 106 verbunden ist und in der die Rate f_{RR} der erfaßten Herzaktionen (Ventrikeldepolarisationen bzw. R-Wellen) ermittelt wird.

Der Ausgang der Zählerstufe 105 ist über einen Meßsignaleingang 107a mit einer Additionsstufe 107 verbunden, und es ist ein Inkrement-/Dekrementspeicher 108 für additiv oder subtraktiv zu berücksichtigende (d.h. positive oder negative) Herzraten-Korrekturwerte vorgesehen. Unter Zeitsteuerung bzw. Taktung durch den Zeitgeber 106 werden aus dem Speicher 108 nach dem Zufallsprinzip gespeicherte Korrekturwerte für den aktuellen Wert der Herzrate fRR über deren zweiten Eingang 107b in die Additionsstufe 107 gelesen und dort zur Korrektur des über deren ersten Eingang 107a zugeführten aktuellen Meßwerts eingesetzt. Die Summe oder Differenz (bzw. zwischenzeitlich auch jeweils der unkorrigierte Meßwert) wird am Ausgang 107c ausgegeben.

Es wird hier angenommen, daß der Speicher 108 über eine Anzahl von Speicherbereichen 108a bis 108n verfügt, in denen Inkrement-/Dekrementwerte innerhalb eines Streuungsbereiches von beispielsweise +/-25 ms gespeichert sind, und daß deren Adressierung auf statistische Weise über einen Zufallsgenerator 109.1 erfolgt. Der Streuungsbereich der Inkrement-/Dekrementwerte kann beispielsweise auch als Bruchteil des gemessenen RR-Intervalls definiert oder patientenspezifisch programmiert werden; wichtig ist jedoch, daß grundsätzlich die Steuerung der Therapie unter Bezugnahme auf einen konkret im bzw. am Körper erfaßten Meßwert erfolgt. Insbesondere gewährleistet der Einsatz eines Zufallsgenerators, wenn die adressierbaren Inkrement- und Dekrementwerte im Streuungsbereich gleichmäßig verteilt sind, daß der zeitliche Mittelwert des Korrekturbetrages Null ist.

Der Ausgang 107c der Additionsstufe 107 ist über einen Signaleingang 110a einer Therapie-Vorwahleinheit 110 einerseits mit einem Steuereingang einer Schrittmacherstufe 111 und andererseits mit einem Steuereingang einer Kardioverterstufe 112 verbunden, so daß der am Ausgang der Stufe 107 anliegende RR-Intervall-Meßwert oder -Summenwert entweder zur Ansteuerung eines Schrittmachers zur Ausgabe anitbradykarder oder antitachykarder Impulsfolgen oder aber zur Auslösung eines hochenergetischen Schockimpulses (Defibrillationsoder Kardioversionsimpulses) dienen kann.

Der Ausgang der Schrittmacherstufe 111 ist über die Elektrodenleitung 102 mit der Ventrikelelektrode 103 verbunden, die somit eine Doppelfunktion als Abfühlelektrode des Kombinationsgerätes 101 und als Stimulationselektrode seiner Schrittmacherstufe 111 hat. Der Ausgang der Kardioverterstufe 112 ist über eine zweite, intrathorakal verlegte Elektrodenleitung 113 mit einer epikardial am Herzen H angeordneten Defibrillations-Flächenelektrode 114 verbunden, über die im Bedarfsfall die hohe Energie eines Defibrillationsschocks auf das reizbare Herzgewebe mit noch gewebsverträglicher Energiedichte übertragen wird.

Die Therapie-Vorwahleinheit 110 umfaßt einen Komparator 110.1, der über einen ersten Dateneingang mit der Additionsstufe 107 und über einen zweiten Dateneingang 110b mit zwei separat adressierbaren Speicherbereichen 115a, 115b eines Bereichsgrenzenspeichers 115 verbunden ist. In diesen sind die oben unter Bezugnahme auf Fig. 1 erwähnten beiden Grenzen tf und tt3 der Bereiche VF und VT2 im RR-Intervall- bzw. Herzratenkontinuum als patientenspezifisch vorprogrammierte Werte gespeichert. Dem Bereichsgrenzenspeicher 115 ist ein Adreßwähler bzw. speicher 116 zugeordnet, der - wie weiter unten genauer erläutert wird - in Abhängigkeit von einem die Therapie-Vorgeschichte reflektierenden internen Steuersignal jeweils einen der beiden Speicherbereiche 115a, 115b mit dem Komparator 110.1 verbindet, den anderen aber sperrt. Der Komparator 110.1 ist ausgangsseitig mit einem Umschalter 110.2 verbunden, der in Abhängigkeit vom Ergebnis des Vergleiches des aktuellen Ausgangswertes der Additionsstufe 107 mit dem gespeicherten Bereichsgrenzwert tf oder tt3 die Schrittmacherstufe 111 oder die Kardioverterstufe 112 aktiviert und ggfs. zugleich den besagten Ausgangswert zur Schrittmacherstufe 111 durchschaltet.

Die Schrittmacherstufe 111 weist eine mehrstufige Vergleichereinheit 111.1 auf, die - in ähnlicher Weise wie die Therapie-Vorwahleinheit 110 - über zweite Eingänge mit Speicherbereichen 115c bis 115h des Bereichsgrenzenspeichers 115 verbunden ist, wobei auch hier wieder in Abhängigkeit von der Therapie-Vorgeschichte über den Adreßwähler 116 eine Adressierung oder aber Sperrung jeweils eines der Bereiche 115c (Wert tt2) oder 115d (Wert tt1), 115e (Wert ts2) oder 115f (Wert ts1) bzw. 115g (Wert sb2) oder 115h (Wert sb1) erfolgt. In der mehrstufigen Vergleichereinheit 111.1 wird der Ausgangswert der Additionsstufe 107 sequentiell mit den verschiedenen Bereichsgrenzen verglichen und in Abhängigkeit vom Vergleichsergebnis am Ausgang der Vergleichereinheit 111.1 ein Signal ausgegeben, das die Zuordnung des Ausgangssignals der Stufe 107 zu einem der o.g. Herzraten- bzw. RR-Intervallbereiche ausdrückt.

Dieses Signal wird zur Adressierung einem ImpulsfolgenSteuersignalspeicher 111.2 zugeführt, woraufhin aus dem jeweils adressierten Speicherplatz ein vorgespeichertes Impulsfolgemuster (das sich insbesondere durch eine vorbestimmte Stimulationsrate, aber ggfs. auch durch einen bestimmten Aufbau und weitere Parameter auszeichnet) an einen Schrittmacher-Impulsgenerator 111.3 ausgegeben wird. Dieser erzeugt eine dem Impulsfolgemuster entsprechende Folge von Impulsen, die in üblicher Weise eine Ausgangsstufe 111.4 durchlaufen und über die Elektrode 103 an das Herz H abgegeben werden, falls die Schrittmacherstufe 111 aktiviert ist. Dies ist nach obigem genau dann der Fall, wenn das Ausgangssignal der Stufe 307 einem der Bereiche VT2, VT1 oder Brady zuzuordnen ist.

Ist hingegen im Falle eines im Ratenbereich VF liegenden Ausgangsignals der Stufe 107 die Kardioverterstufe 112 aktiviert, wird dort (in an sich bekannter Weise) ein einzelner Schockimpuls mit vorprogrammierten Parametern erzeugt und über die Elektrode 114 an das Herz H abgegeben.

Sowohl der Ausgang des Komparators 110.1 in der Therapie-Vorwahleinheit 110 als auch derjenige der mehrstufigen Vergleichereinheit 111.1 sind zusätzlich mit einem Meßwertspeicher 117 verbunden, der bei jeder - in den Vergleicherstufen 110.1 oder 111.1 ermittelten - Änderung der Zuordnung des Ausgangswertes der Additionsstufe 107 zu einem der oben erwähnten Bereiche des Herzraten- bzw. RR-Intervall-Kontinuums zur Einspeicherung der jeweils aktuellen Zuordnung adressiert wird und in dem somit eine vorbestimmmte Anzahl von (in gewissem Sinne die Therapie-Vorgeschichte reflektierenden) Zuordnungen der Herzrate bzw: des RR-Intervalls aus der Vergangenheit gespeichert wird. Einfachstenfalls wird nur die Zuordnung vor der jeweils letzten Änderung gespeichert, in einer aufwendigeren, nach dem LI-FO(last-in-first-out)-Prinzip organisierten, Ausführung mit mehreren Speicherbereichen arbeitet der Speicher 117 als regelrechter Trendspeicher.

Der Meßwertspeicher 117 ist mit einem Eingang einer Trend-Auswertungsstufe 118 verbunden, die über einen weiteren Eingang zudem mit dem Ausgang der Additionsstufe 107 verbunden ist und deren Ausgang mit dem Adreßwähler 116 in Verbindung steht. Die Trend-Auswertungsstufe 118 liefert im Ergebnis der Auswertung der zeitlichen Entwicklung der Herzraten- bzw. RR-Intervall-Zuordnung - ausgehend vom aktuellen Wert mindestens über eine Änderungsstufe hinweg - das oben erwähnte Steuersignal für den Adreßwähler bzw. Pointer 116. Über diesen wird (gemäß obiger Erläuterung) wiederum die in den Überlappungsregionen tf-tt3, tt2-tt1, ts2-ts1 und sb2-sb1 aktuell gültige Bereichszuordnung des Ausgangssignals der Stufe 107 und damit weiter die gültige Therapie (Defibrillationsschock oder konkret anzuwendende Antitachykardie- oder Bedarfsstimulations-Impulsfolge) bestimmt.

Nachfolgend soll das Zusammenwirken der vorstehend beschriebenen Funktionseinheiten nach Fig. 2, insbesondere hinsichtlich der Therapiesteuerung in den Überlappungsregionen, anhand spezieller Situationen erläutert werden, die bei einem zu bestimmten tachykarden Rhythmusstörungen disponierten Patienten auftreten können, bei dem die Herzrate bzw. das RR-Intervall in die Bereiche VT1, VT2 oder VF fallen kann:

Grundsätzlich ist jedem der Bereiche über der RR-Achse in Fig. 1 eine spezifische Elektrostimulations-Therapie (d.h. ein Satz von Stimulationsparametern) zugeordnet - wobei zu beachten ist, daß unter "RR-Rate" bzw. "RR-Intervall" ein mit einem Schwankungs-Inkrement bzw. -Dekrement korrigierter Meßwert verstanden werden soll. Ändert sich der mit der Schwankungsgröße behaftete Meßwert (das Ausgangssignal der Additionsstufe 107) innerhalb eines Bereiches, wird die Therapie nicht geändert. Überschreitet er jedoch eine Bereichsgrenze, schaltet der in Fig. 2 schematisch dargestellte Schrittmacher/Kardioverter grundsätzlich auf eine andere einer Mehrzahl vorbestimmter Therapien um. Infolge der Behaftung mit der statistischen Schwankungsgröße erfolgt bei Meßwerten, die in den grenznahen Gebieten der Bereiche liegen, gelegentlich ein Umschalten zwischen verschiedenen Therapien, mit anderen Worten: die Schwankungsgröße ermöglicht ein "Ausprobieren" verschiedener Therapien bzw. Therapiegrößen.

Kommt nun bei der vorliegenden Ausführung beispielsweise im Ergebnis einer Beschleunigung einer Tachykardie die (schwankungsbehaftete) Herzrate vom VT1-Bereich in die Überlappungsregion zum VT2-Bereich zwischen den Grenzwerten tt1 und tt2, wird auf die für den Bereich VT2 gültige ("aggressivere") Therapie umgeschaltet. Dies ist therapeutisch folgerichtig, da die im Bereich VT1 angewandte (weniger "aggressive") Therapie die Beschleunigung der Tachykardie nicht verhindern konnte, sich mithin als zu schwach erwies. Gelangt die Herzrate im Zuge der Verlangsamung einer ursprünglich im VT2-Bereich liegenden Tachykardie ebenfalls in die Überlappungsregion tt1-tt2, wird die für den VT2-Bereich gültige Therapie beibehalten. Auch dies ist folgerichtig, denn diese Therapie hat sich als erfolgreich erwiesen.

Springt hingegen die Herzrate aus dem Bereich Sinus in dieselbe Überlappungsregion tt2-tt1 der Bereiche VT1, VT2, so wird nicht die für den Bereich VT2, sondern die für den Bereich VT1 gültige Therapie (Impulsfolge) eingeschaltet. Dieses Vorgehen beruht auf der Überlegung, daß bei dieser Entwicklung der Körper des Patienten nicht übergangslos mit der "aggressiveren" Therapie für den Bereich VT2 belastet werden sollte, solange nicht versuchsweise die für den Bereich VT1 gültige Therapie angewandt worden ist. Damit soll auch das Risiko minimiert werden, durch die Therapie selbst eine weitere Beschleunigung der Tachykardie zu induzieren. Die für den Bereich VT2 gültige Therapie wird erst angewandt, wenn sich die Herzrate derart weiter erhöht, daß sie die Überlappungsregion verläßt.

Entsprechend differenziert wird die gültige Therapie auch bei einem entweder langsamen oder aber sprunghaften Übergang der Herzrate in die Überlappungsregion tf-tt3 zwischen den Bereichen VT2 und VF gewählt. Die Überlappungsregionen ts1-ts2 zwischen den Bereichen Sinus und VT1 und sb1-sb2 zwischen den Bereichen Brady und Sinus könen jedoch als "klassische" Hysteresebereiche behandelt werden, in denen mithin die Wahl der Therapie lediglich von der Richtung des Eintritts in die Überlappungsregion abhängt.

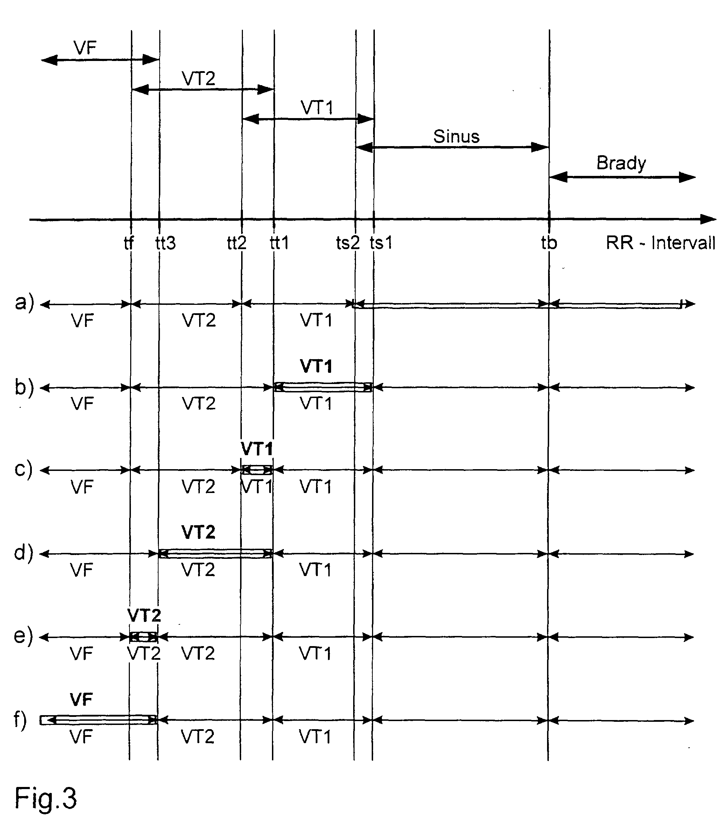

Die oben skizzierte Betriebsweise des Schrittmachers/Kardioverters gemäß der vorliegenden Ausführungsform ist in Fig. 3 illustriert, bei der die Bereichsunterteilung im wesentlichen Fig. 1 entspricht (lediglich der Überlappungsbereich sb1-sb2 zwischen den Bereichen Brady und Sinus ist hier weggelassen):
- Zeile a) zeigt, daß, wenn sich in einer ersten Phase die Herzrate in einem der Bereiche Brady oder Sinus befindet und keine Therapie bzw. eine Bradykardie-Stimulation angewandt wird, bei einer anschließenden Beschleunigung in den Raten-Bereich zwischen ts2 und tt1 die Therapie "VT1", zwischen tt2 und tf die Therapie "VT2" und oberhalb von tf die Therapie "VF" angewandt wird.
- Zeile b) zeigt, daß, wenn anfänglich die Rate im Gebiet zwischen ts1 und tt1 war und mit VT1 therapiert wurde (was für RR-Werte zwischen ts1 und ts2 nach obigem übrigens nur dann zutrifft, wenn diese Region im Zuge der Verlangsamung einer Tachykardie erreicht worden war), ein anschließender Übergang in das Gebiet zwischen tt1 und tf zur Anwendung der Therapie "VT2", ein Übergang auf einen Wert oberhalb tf aber zur Anwendung der Therapie "VF" führt. Eine Verlangsamung in den Bereich zwischen tb und ts1 hinein führt zum Aussetzen jeglicher Therapie und eine solche unter tb zu einer Bradykardie-Stimulation was auch für die folgenden Zeilen gilt.
- Zeile c) zeigt, daß, wenn anfänglich die Rate im Gebiet zwischen tt1 und tt2 war und entsprechend die Therapie "VT1" angewandt worden war (was nach obigem nur dann der Fall sein wird, wenn die Region im Zuge einer sich schnell beschleunigenden Tachykardie erreicht wurde), ein anschließender Übergang in das Gebiet zwischen tt2 und tf zur Anwendung der Therapie "VT2", ein Übergang auf einen Wert oberhalb tf zur Anwendung der Therapie "VF", eine Verlangsamung der Tachykardie in das Gebiet zwischen ts1 und tt1 aber zur Beibehaltung der Therapie "VT1" führt.
- Zeile d) zeigt, daß, wenn anfänglich die Rate im Gebiet zwischen tt1 und tt3 war und die Therapie "VT2" angewandt worden war, ein anschließender Übergang auf einen Wert oberhalb tt3 zur Anwendung der Therapie "VF" führt. Eine Verlangsamung in den Bereich zwischen ts1 und tt1 hinein führt zur Umschaltung auf die Therapie "VT1". Letzteres gilt auch für die Zeilen e) und f).
- Zeile e) zeigt, daß, wenn anfänglich die Rate im Gebiet zwischen tt3 und tf war und entsprechend die Therapie "VT2" angewandt worden war, ein anschließender Übergang auf einen Wert oberhalb tf zur Anwendung der Therapie "VF", eine Verlangsamung in das Gebiet zwischen tt1 und tt3 jedoch zur Beibehaltung der Therapie "VT2" führt.
- Zeile f) schließlich zeigt, daß ausgehend von einer Rate oberhalb von tt3, bei der die Therapie VF angewandt wurde, eine Verlangsamung in das Gebiet zwischen tt1 und tt3 hinein zur Umschaltung auf die Therapie "VT2" führt.

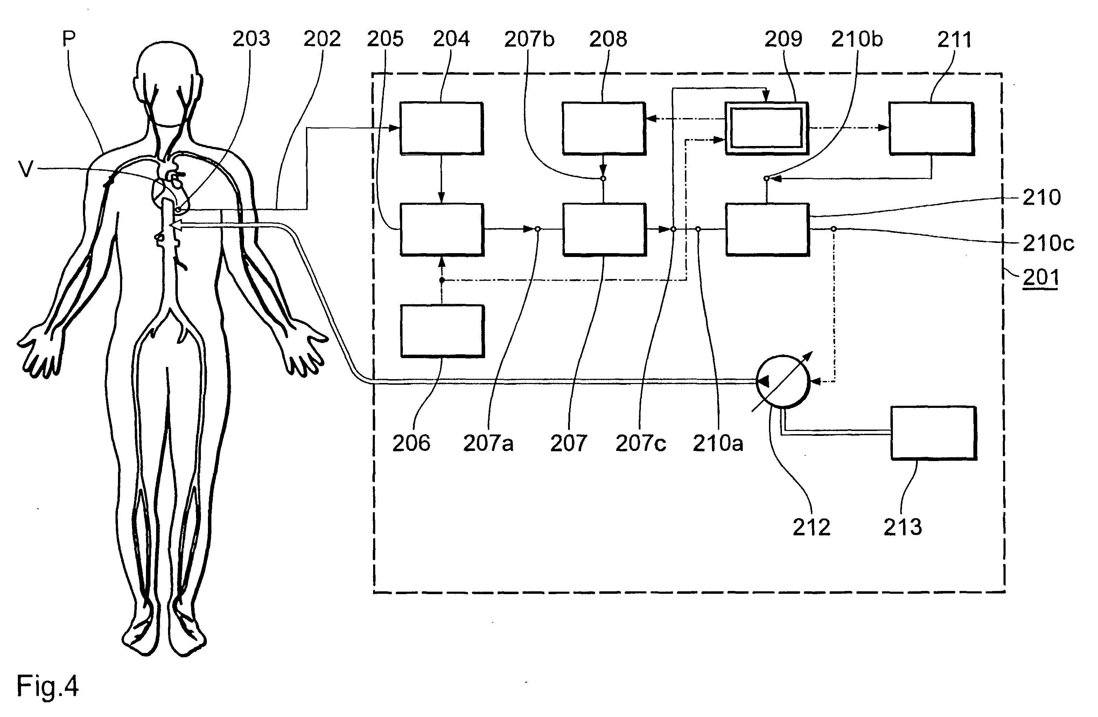

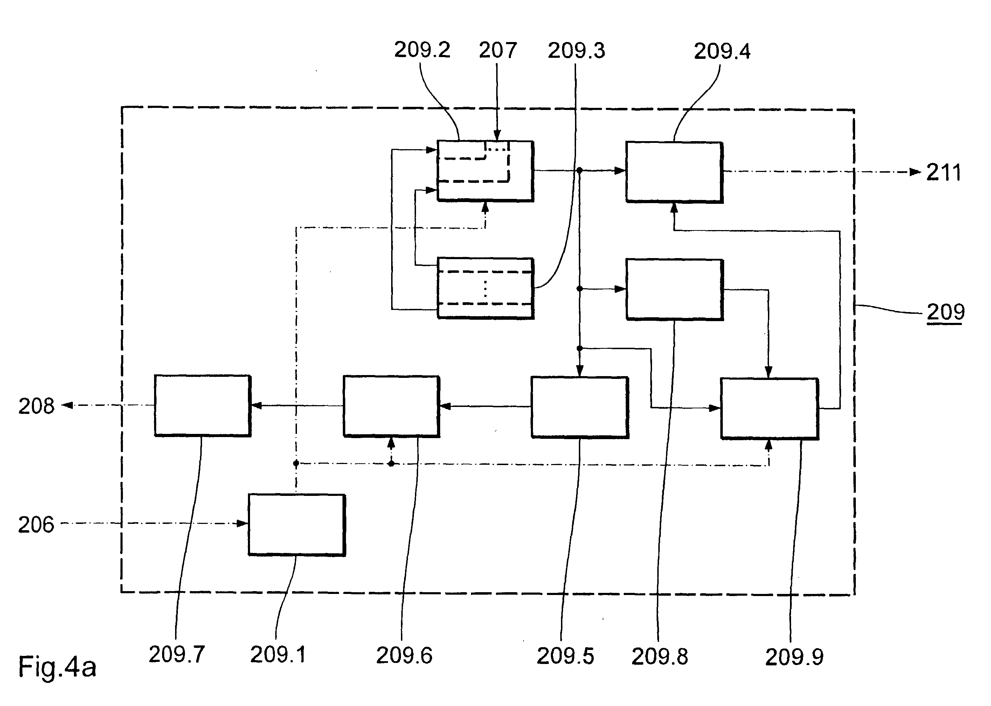

Figur 4 ist ein - wie schon die Fig. 2 - stark vereinfachtes Funktions-Blockschaltbild eines Medikamentendosiergerätes 201, speziell für Medikamente zur Korrektur von Herzarrhythmien. Die nachfolgend beschriebene Ausführung eignet sich grundsätzlich zur Steuerung einer selbsttätigen medikamentösen Behandlung von plötzlich auftretenden lebensbedrohlichen Bradykardien oder Tachykardien mittels geeigneter Antiarrhythmika.

Das Gerät 201 ist über eine Elektrodenleitung 202 mit einer im Ventrikel V eines Herzens H positionierten intrakardialen Herzelektrode 203 zur Aufnahme von Herzaktionspotentialen verbunden. Das proximale Ende der Elektrodenleitung 202 ist mit einer Eingangsverstärkerstufe 204 verbunden, die (in an sich von Herzschrittmachern bekanntem Aufbau) Filter- und Verstärkerstufen zur Signalaufbereitung aufweist und an deren Ausgang ein weitgehend störbefreites, pegelangepaßtes Herzsignal anliegt. Der Ausgang der Eingangsstufe 204 ist mit dem Signaleingang einer Zählerstufe 205 verbunden, deren Takteingang mit einem Takt-bzw. Zeitgeber 206 verbunden ist und in der die Rate f_{RR} der erfaßten Herzaktionen (Ventrikeldepolarisationen bzw. R-Wellen) ermittelt wird.

Ähnlich wie bei der Anordnung nach Fig. 2, ist der Ausgang der Zählerstufe 205 über einen Meßsignaleingang 207a mit einer Additionsstufe 207 verbunden, und es ist ein Inkrement-/Dekrementspeicher 208 für additiv oder subtraktiv zu berücksichtigende (d.h. positive oder negative) Herzraten-Korrekturwerte vorgesehen. Unter Steuerung durch eine Programmsteuereinheit 209, die mit dem Taktgeber 206 verbunden ist und weiter unten genauer erläutert wird, wird eine grundsätzlich der Anordnung nach Fig. 2 entsprechende (und daher hier nicht nochmals erläuterte) SchwankungswertVerarbeitung der gemessenen Herzrate ausgeführt.

Der Ausgang 207c ist mit einem ersten Signaleingang 210a einer Auswertungs- und Pumpenansteuereinheit 210 verbunden, die über einen zweiten Signaleingang 210b mit einem Dosierdatenspeicher 211 in Verbindung steht. Aufbau und Organisation dieses Speichers entsprechen im wesentlichen denen des Speichers 111.2 aus Fig. 2, wobei im vorliegenden Beispiel eine Zuordnungstabelle Herzratenbereich Medikamentendosis gespeichert ist. Der jeweils aus dem adressierten Speicherplatz ausgelesene Dosiswert wird der Ermittlung einer zugehörigen geräteinternen Steuergröße zugrundegelegt, die am Ausgang 210c der Auswertungsund Pumpenansteuereinheit 210 bereitgestellt wird. Dieser ist mit einer Dosierpumpe 212 verbunden, über die ein in einem Medikamententank 213 gespeichertes Antiarrhythmikum mit der berechneten Dosierung in den Körper des Patienten P abgegeben werden kann. Bei einem gefährlichen Abfallen oder Ansteigen der Herzrate in einen kritischen Bereich (je nach Einsatz des Gerätes zur Therapie bradykarder oder tachykarder Rhythmusstörungen) wird somit automatisch eine medikamentöse Behandlung eingeleitet, deren Erfolg anhand der über die Herzelektrode 203 aufgenommenen Signale kontrollierbar ist.

Die sich entsprechend der Wirkung des injizierten Medikamentes ergebende, über die Elektrode 203, die Eingangsstufe 204 und die Zählerstufe 205 erfaßte, aktuelle Herzrate wird wieder der oben beschriebenen Verarbeitung unterzogen. Beim vorliegenden Beispiel ist zu beachten, daß das Dosiergerät 201 eine Notfalltherapie bewirken soll, d.h. die Dosierpumpe 212 im Normalfall außer Betrieb sein soll. Entsprechend sind auch die vorgespeicherten Inkrement-bzw. Dekrementwerte und die Zuordnungstabelle für Herzrate und Medikamentendosis in Abstimmung aufeinander derart zu wählen, daß eine Medikamentenabgabe nur bei gemessenen wie auch korrekturbehafteten Werten der Herzrate erfolgt, die einem für den Patienten mit hoher Wahrscheinlichkeit kritischen Bereich zuzuordnen sind.

Eine Erfassung und Speicherung eines therapeutisch vorteilhaften Inkrement- oder Dekrementwertes ist hier verzichtbar, da es bei der Notfalltherapie weniger darauf ankommt, ob der ursprüngliche oder einer (und welcher) der variierten Herzraten-Meßwerte der Ausgangspunkt für eine wirksame medikamentöse Behandlung war, als vielmehr entscheidend darauf, daß diese effizient war. Zudem können sich bis zu einem eventuellen erneuten Bradykardie- oder Tachykardienanfall die patientenseitigen Bedingungen erheblich ändern, so daß die Variation der Therapiesteuerung dann ggfs. zweckmäßigerweise vom gleichen "neutralen" Ausgangspunkt - nämlich dem aktuellen Meßwert - aus erfolgen sollte.

In Fig. 4a ist in einem vereinfachten Funktions-Blockschaltbild eine Ausführungsform der Auswertungs- und Steuereinheit 209 des Medikamentendosiergerätes 201 nach Fig. 4 gezeigt. Es ist zu beachten, daß diese - wie auch im wesentlichen die Funktionsgruppen zur Steuerung des in Fig. 2 gezeigten Gerätes - in der Praxis im wesentlichen als Mikrocontroller mit enger Verknüpfung zwischen hard-und softwaremäßiger Realisierung ausgeführt werden wird.

Die Auswertungs- und Steuereinheit umfaßt eine interne Ablaufsteuerung 209.1, die über den Zeitgeber 206 getaktet wird und ihrerseits zunächst periodisch eine mehrstufige Vergleichereinheit 209.2 aktiviert, die über einen Eingang mit dem Ausgang der Additionsstufe 207 verbunden und über einen weiteren Eingang sequentiell mit den Speicherbereichen eines als EPROM ausgeführten Meßbereichsspeichers 209.3 verbindbar ist. Die Vergleichereinheit 209.2 ist ausgangsseitig zum einen mit einem Adreßzeiger 209.4 verbunden, in dem das Ausgangssignal der Vergleichereinheit in ein Adreßsignal für den Dosierwertspeicher 211 umgesetzt wird. Andererseits ist sie mit einem Digitalfilter 209.5 verbunden, das nur vorgegebene Ausgangssignale der Vergleichereinheit zu einem Eingang eines UND-Gatters 209.6 gelangen läßt, an dessen anderem Eingang das Taktsignal der Ablaufsteuerung 209.1 anliegt. Der Ausgang des UND-Gatters 209.6 ist mit einem Zufallsgenerator 209.7 verbunden, an dessen Ausgang schließlich ein Adreßsignal für den Inkrement-/Dekrementspeicher 208 bereitsteht.

Der Ausgang der Vergleichereinheit 209.2 ist weiterhin mit einem Bereichszuordnungsspeicher 209.8 sowie einer Zuordnungs-Vergleichereinheit 209.9 verbunden, die über ihren zweiten Eingang mit dem Bereichszuordnungsspeicher und ausgangsseitig mit dem Adreßwähler 209.4 verbunden ist.

In der Vergleichereinheit 209.2 werden die an ihrem einen Eingang anliegenden (potentiell korrigierten, d.h. periodisch schwankungswertbehafteten) Herzratenwerte sequentiell mit den vorprogrammierten, im Bereichsgrenzenspeicher gespeicherten Grenzwerten mehrerer Herzratenbereiche mit jeweils spezifischer therapeutischer Relevanz verglichen und die Zugehörigkeit zu einem der Bereiche bestimmt. Diese wird über die Art des Ausgangssignals der Vergleichereinheit 209.2 zum Ausdruck gebracht, welches einerseits direkt dem Adreßwähler bzw. -zeiger 209.3 und andererseits zum Vergleich der aktuellen mit der vorhergehenden Wertebereichszuordnung des Meßwertes der Zuordnungs-Vergleichereinheit 209.9 zugeführt wird. Die Ausgänge beider Komparatoren 209.2 und 209.9 adressieren den Adreßwähler 209.3, der beispielsweise eine zweidimensionale Zuordnungstabelle von Dosierwertspeicheradressen zu Ausgangswertepaaren der beiden Komparatoren enthält. Hiermit wird - in ähnlicher Weise wie oben unter Bezugnahme auf Fig. 2 erläutert - die Auswahl einer konkret anzuwendenden Medikamentendosierung aus zwei möglichen Werten in Überlappungszonen zwischen zwei Herzratenbereichen in Abhängigkeit von der vorhergehenden Bereichszuordnung der Herzrate (d.h. zugleich von der vorhergehenden Therapie) realisiert. Der Adreßwähler bewirkt nach Maßgabe dieser Tabelle die Adressierung eines bestimmten Bereiches des Dosierwertspeichers 211, d.h. die Ausgabe einer bestimmten Steuergröße an die Dosierpumpe.

Die Schwankungswert-Verarbeitung der Meßgröße geschieht bei der gezeigten Anordnung ebenfalls ausgehend vom Ausgangsignal der Vergleichereinheit 209.2. Dieses bestimmt nämlich, indem es - je nach Signalcharakteristik - das Filter 209.5 passiert oder nicht passiert und über das UND-Gatter 209.6 die Ausgabe eines Ansteuersignals an den Zufallsgenerator 209.7 durch die Ablaufsteuerung 209.1 freigibt oder sperrt, ob der gemessenen Herzrate ein Schwankungswert aufgeprägt wird oder nicht.

Die Erfindung beschränkt sich in ihrer Ausführung nicht auf die vorstehend angegebenen bevorzugten Ausführungsbeispiele. Vielmehr ist eine Anzahl weiterer Varianten denkbar, welche von der dargestellten Lösung Gebrauch macht.

So ist - nachdem oben die Erfassung einer vorhergehend angewandten Therapie über die Erfassung der vorhergehenden Wertebereichzuordnung der (evtl. korrigierten) Meßgröße beschrieben wurde - , anzumerken, daß stattdessen auch die vorhergehende Therapiesteuergröße oder sogar die Therapiegröße selbst gespeichert und mit der aufgrund der aktuellen Bereichszuordnung der Meßgröße anzuwendenden Therapiesteuergröße bzw. Therapiegröße verglichen werden kann.

Die praktische Realisierung der Funktionsblöcke der in den Figuren dargestellten Anordnungen ebenso wie die Wahl geeigneter Parameter zur Ausführung der einzelnen Therapien, etwa als spezifische Stimulationsimpulsfolgen (bzw. der Therapie "VF" als Defibrillationsschock), liegt im Rahmen fachmännischen Handelns und bedarf daher hier keiner genaueren Erläuterung. Es ist darauf hinzuweisen, daß auch eine Realisierung der Funktionselemente im Rahmen einer Mikroprozessorsteuerung - auch softwaremäßig - im Rahmen der in den Ansprüchen definierten Erfindung liegt.

## Patentansprüche

1. Therapiegerät, insbesondere implantierbarer Herzschrittmacher, Kardioverter, kombinierter Herzschrittmacher/Kardioverter (101) oder Medikamentendosiergerät (201), mit mindestens einem Sensor (103; 203) zum Erfassen einer bei Anwendung einer vorbestimmten Therapie im oder am Körper eines Patienten (P) meßbaren, insbesondere dessen körperlichen Zustand kennzeichnenden, Größe und zur Ausgabe eines entsprechenden Meßwertes, einer mindestens mittelbar mit dem Ausgang des Sensors verbundenen Auswertungs- und Steuereinrichtung (110, 111; 209) zur Auswertung des Meßwertes einschließlich seiner Zuordnung zu einem von mindestens zwei vorbestimmten Wertebereichen und zur Bestimmung einer Therapiesteuergröße in Abhängigkeit hiervon und einer mit dem Ausgang der Auswertungs-und Steuereinrichtung verbundenen Therapieeinrichtung (111.3, 111.4, 112; 212), die zur Realisierung verschiedener Therapien oder Therapiegrößen in Abhängigkeit von der Zugehörigkeit des Wertes der Meßgröße zu einem der Wertebereiche ausgebildet ist,
**dadurch gekennzeichnet,**
**daß** die Auswertungs- und Steuereinrichtung
- einen Bereichsgrenzenspeicher (115; 209.3) zur Speicherung mindestens zweier verschiedener Grenzen zwischen zwei Wertebereichen der Meßgröße und damit mindestens einer Überlappungszone zwischen den Wertebereichen,
- eine über einen Eingang mindestens mittelbar mit dem Sensor (103; 203) und über einen weiteren Eingang mit dem Bereichsgrenzenspeicher (115; 209.3) verbundene erste Vergleichereinheit (110.1; 209.2) zur Zuordnung des Meßwertes der Größe zu einem der Wertebereiche,
- einen Therapiespeicher (111.2; 211) mit mindestens zwei separat adressierbaren Speicherbereichen zur Speicherung mindestens zweier verschiedener Werte der Therapiesteuergröße in Zuordnung zu den Werten der Meßgröße innerhalb einer Überlappungszone jeweils zusammen mit einer eine Meßgrößen- oder Therapie-Vorgeschichte repräsentierenden vorgegebenen Bedingungsgröße und weiteren Speicherbereichen zur Speicherung jeweils eines Wertes der Therapiesteuergröße in Zuordnung zu einem Wert der Meßgröße außerhalb einer Überlappungszone,
- einen Vorgeschichte-Speicher (117; 209.8) zur Speicherung der Bereichszuordnung des jeweils vorhergehenden Wertes der Meßgröße oder der Therapiesteuergröße einer vorher angewandten Therapie als Bedingungsgröße und
- Vorgeschichte-Auswertungsmittel (118; 209.9) zur Auswertung des gespeicherten Wertes mit den vorgegebenen Werten der Bedingungsgröße und zur Ausgabe von das Auswertungsergebnis ausdrückenden Adreßdaten an den Therapiespeicher zum Auslesen genau eines Wertes der Therapiesteuergröße
aufweist.

2. Therapiegerät nach Anspruch 1, **dadurch gekennzeichnet, daß** eine Ablaufsteuerung vorgesehen ist, die einen Zeitgeber (106; 206) aufweist derart, daß unter Steuerung durch den Zeitgeber periodisch der aktuelle Wert der Meßgröße abgefragt und mit den gespeicherten Grenzen verglichen und ein das Vergleichsergebnis kennzeichnendes Zugriffssteuer- und Adreßsignal ausgegeben wird, das ein Auslesen aus dem Therapiespeicher (111.2; 211) bewirkt oder unterbindet.

3. Therapiegerät nach Anspruch 1 oder 2 **gekennzeichnet durch** eine Ausbildung der Ablaufsteuerung derart, daß ein Zugriffssteuersignal zum Auslesen aus dem Therapiespeicher (111.2; 211) nach Feststellung der Überschreitung einer Wertebereichsgrenze ausgegeben wird.

4. Therapiegerät nach einem der vorangehenden Ansprüche, **dadurch gekennzeichnet, daß** eine eingangsseitig mit der ersten Vergleichereinheit (110.1; 209.2) und dem Vorgeschichte-Speicher (117; 209.8) sowie ausgangsseitig mit einem dem Bereichsgrenzenspeicher (115) oder Therapiespeicher (111.2; 211) zugeordneten Adreßwähler (116; 209.4) verbundene zweite Vergleichereinheit (118; 209.9) zum Vergleich der aktuellen mit der gespeicherten Wertebereichszuordnung der Größe vorgesehen ist.

5. Therapiegerät nach einem der vorangehenden Ansprüche, **gekennzeichnet durch** die Ausführung als implantierbarer Bedarfsschrittmacher (111) mit einem Bereichsgrenzenspeicher (115), der zur Speicherung mindestens einer Grenze zwischen einem Normal- und einem Bradykardie-Ratenbereich sowie zweier Grenzen und somit einer Überlappungszone zwischen dem Normal- und einem Tachykardie-Ratenbereich, sowie einem Therapiespeicher (111.2), der zur Speicherung mindestens einer Bradykardie- und einer Tachykardie-Therapie ausgebildet ist.

6. Therapiegerät nach einem der vorangehenden Ansprüche, **gekennzeichnet durch** die Ausführung als implantierbares Antitachykardie-/-fibrillationsgerät, insbesondere Antitachykardie-Schrittmacher/Defibrillator (101), mit einem Bereichsgrenzenspeicher (115), der zur Speicherung mindestens einer Grenze zwischen einem Normal-und einem Tachykardie-Ratenbereich sowie zweier Grenzen und somit einer Überlappungszone zwischen dem Tachykardie-Ratenbereich und einem Fibrillationsbereich ausgebildet ist.

7. Therapiegerät nach Anspruch 5 und 6 **gekennzeichnet durch** die Ausbildung des Bereichsgrenzenspeichers (115) mit je zwei Speicherbereichen zur Speicherung je zweier Grenzen zwischen dem Normal- und dem Tachykardie-Ratenbereich sowie dem Tachykardie-Ratenbereich und dem Fibrillationsbereich.

8. Therapiegerät nach Anspruch 6 oder 7, **dadurch gekennzeichnet, daß** der Bereichsgrenzenspeicher (115) Speicherbereiche für zwei, vorzugsweise über eine zwischen zwei Grenzen liegende Überlappungszone aneinander angrenzende, Tachykardie-Ratenbereiche aufweist.

9. Therapiegerät nach einem der vorangehenden Ansprüche, **dadurch gekennzeichnet, daß** der Bereichsgrenzenspeicher (115) zwei Speicherbereiche zur Speicherung zweier Grenzen und somit einer Überlappungszone zwischen dem Normal- und dem Bradykardie-Ratenbereich aufweist.

10. Therapiegerät nach einem der vorangehenden Ansprüche, **dadurch gekennzeichnet, daß** zwischen den Ausgang des Sensors (103; 203) und den Eingang der Auswertungs- und Steuereinrichtung (110, 111.1, 111.2, 115; 209) eine eingangsseitig mit dem Ausgang eines Schwankungswertgenerators (108, 108.1; 208, 209.7) verbundene mathematische Verarbeitungseinheit (107; 207) geschaltet ist, in der unter Steuerung durch eine Zeitsteuereinheit (106; 206) dem Sensor-Meßwert zu mindestens einem Zeitpunkt mindestens ein Schwankungswert aufgeprägt wird derart, daß nach Auswertung des mit dem oder einem der Schwankungswert(e) korrigierten Meßwertes ein gegenüber der Auswertung des ursprünglichen Meßwertes und/oder des mit einem anderen Schwankungswert korrigierten Meßwertes veränderter Wert der Therapiesteuergröße erhalten wird.

11. Therapiegerät nach einem der vorangehenden Ansprüche, **dadurch gekennzeichnet, daß** der Sensor (103; 203) zur Erfassung einer Aktivitätsgröße oder einer eine Organfunktion des Patienten (P) kennzeichnenden Größe ausgebildet ist.

12. Therapiegerät nach Anspruch 11 **gekennzeichnet durch** die Ausbildung des bzw. des ersten Sensors als intrakardiale Elektrode (203) mit nachgeschaltetem Abfühlverstärker (204) für elektrische Aktivität des Herzens mit zugeordneter Einrichtung zur Bestimmung der Periode der elektrischen Herzaktivität als Meßwert.

13. Therapiegerät nach einem der vorangehenden Ansprüche, **dadurch gekennzeichnet, daß** der Sensor (103) zur Erfassung der Therapiegröße ausgebildet ist.

14. Therapiegerät nach einem der Ansprüche 10 bis 13, **dadurch gekennzeichnet, daß** der Schwankungswertgenerator einen Schwankungswertspeicher (108) für mehrere Schwankungswerte und wahlweise einen Zufallsgenerator (108.1) zur Auswahl je eines der gespeicherten Schwankungswerte zur Aufprägung auf den Meßwert aufweist.

15. Therapiegerät nach einem der Ansprüche 1 bis 4 oder 7 bis 14, **gekennzeichnet durch** die Ausbildung als, insbesondere implantierbares, Medikamentendosiergerät (201) derart, daß der bzw. der erste Sensor (203) zur Erfassung des Pegels eines Wirkstoffes oder einer davon abhängigen Größe im Körper des Patienten (P) als Meßwert, die Auswertungs- und Steuereinrichtung (209, 210) zur Festlegung einer Medikamentendosis pro Zeiteinheit und die Therapieeinrichtung (212) zur Abgabe der festgelegten Dosis pro Zeiteinheit an den Körper ausgestaltet sind.

## Claims

1. Therapy device, in particular an implantable cardiac pacemaker, cardioverter, combined pacemaker/cardioverter (101) or medication-dosing device (201), comprising at least one sensor (103; 203) for detecting a variable that can be measured in the application of a predetermined therapy in or on the body of a patient (P) and that in particular characterises the physical state of the patient, and for outputting a corresponding measured value, an evaluating and control device (110, 111; 209) at least indirectly connected to the output of the sensor for evaluating the measured value, including its association with one of at least two predetermined value ranges and for determining a therapy control variable as a function thereof, and further having a therapy device (111.3, 111.4, 112; 212), connected to the output of the evaluating and control device, which is configured for implementing different therapies or therapy variables as a function of the association of the value of the measured variable with one of the value ranges, **characterised in that** the evaluating and control device comprises
- a range-limit memory (115; 209.3) for storing at least two different limits between two value ranges of the measured variable, and thus at least one overlap zone between the value ranges,
- a first comparator unit (110.1; 209.2) that is at least indirectly connected via an input to the sensor (103; 203) and, via a further input, to the range-limit memory (115; 209.3) for associating the measured value of the variable with one of the value ranges,
- a therapy memory (111.2; 211) having at least two separately-addressable memory regions for storing at least two different values of the therapy-control variable in association with the values of the measured variable within an overlap zone, each together with a predetermined conditional variable that represents the past history of a measured variable or the therapy, and further memory regions for respectively storing a value of the therapy-control variable, in association with a value of the measured variable, outside an overlap zone,
- a past-history memory (117; 209.8) for storing the range association of the respectively previous value of the measured variable or of the therapy-control variable of a previously-applied therapy as a conditional variable, and
- past-history evaluation means (118; 209.9) for evaluating the stored value with the predetermined values of the conditional variable, and for outputting address data that express the evaluation result to the therapy memory for precisely reading out a value of the therapy-control variable.

2. Therapy device according to claim 1, **characterised in that** a process control is provided that has a timer (106; 206) such that, with control by the timer, the current value of the measured variable is periodically queried and compared to the stored limits, and an access-control and address signal that effects or prevents a read-out from the therapy memory (111.2, 211) and characterises the comparison result is output.

3. Therapy device according to claim 1 or 2, **characterised by** a configuration of the process control such that an access-control signal is output for a read-out from the therapy memory (111.2; 211) after it has been determined that a value-range limit has been exceeded.

4. Therapy device according to any one of the preceding claims, **characterised in that** a second comparator unit (118; 209.9) that is connected, on the input side, to the first comparator unit (110.1; 209.2) and the past-history memory (117; 209.8) and, on the output side, to an address selector (116; 209.4) associated with the range-limit memory (115) or therapy memory (111.2; 211) is provided for comparing the current value-range association of the variable with the stored one.

5. Therapy device according to any one of the preceding claims, **characterised by** its configuration as an implantable demand pacemaker (111) having a range-limit memory (115), which is configured to store at least one limit between a normal rate range and a bradycardic rate range and two limits and therefore an overlap zone between the normal rate range and a tachycardiac rate range, and a therapy memory (111.2) configured to store at least one bradycardic therapy and one tachycardiac therapy.

6. Therapy device according to any one of the preceding claims, **characterised by** its configuration as an implantable anti-tachycardia/anti-fibrillation device, in particular an anti-tachycardia pacemaker/defibrillator (101), having a range-limit memory (115) that is configured to store at least one limit between a normal rate range and a tachycardia rate range, and two limits and thus an overlap zone between the tachycardia rate range and a fibrillation range.

7. Therapy device according to claim 5 and 6, **characterised by** the configuration of the range-limit memory (115) to have two memory regions for respectively storing two limits between the normal rate range and the tachycardia rate range and between the tachycardia rate-range and the fibrillation range.

8. Therapy device according to claim 6 or 7, **characterised in that** the range-limit memory (115) has memory regions for two tachycardia rate ranges, preferably mutually adjacent over an overlap zone located between two limits.

9. Therapy device according to any one of the preceding claims, **characterised in that** the range-limit memory (115) has two memory regions for storing two limits and thus an overlap zone between the normal rate range and the bradycardia rate range.

10. Therapy device according to any one of the preceding calims, **characterised in that** a mathematical processing unit (107; 207) connected on the input side to the output of a fluctuation-value generator (108, 108.1; 208, 209.7) is connected between the output of the sensor (103; 203) and the input of the evaluating and control device (110, 111.1, 111.2, 115; 209), with at least one fluctuation value being impressed onto the sensor measured value at at least one instant in the processing unit, with control by a time-control unit (106; 206), such that, after evaluation of the measured value corrected with the fluctuation value or one of the fluctuation values, a value of the therapy control variable that is changed in comparison to the evaluation of the original measured value and/or the measured value corrected with a different fluctuation value is obtained.

11. Therapy device according to any one of the preceding claims, **characterised in that** the sensor (103; 203) is configured to detect an activity variable or a variable that characterises an organ function of the patient (P).

12. Therapy device according to claim 11, **characterised by** the configuration of the sensor or the first sensor as an intracardiac electrode (203) having a downstream sensor amplifier (204) for electrical activity of the heart, the device having an associated device for determining the period of electrical cardiac activity as a measured value.

13. Therapy device according to any one of the preceding claims, **characterised in that** the sensor (103) is configured to detect the therapy variable.

14. Therapy device according to any one of claims 10 to 13, **characterised in that** the fluctuation-value generator comprises a fluctuation-value memory (108) for a plurality of fluctuation values and optionally comprises a random-number generator (108.1) for respectively selecting one of the stored fluctuation values for impression onto the measured value.

15. Therapy device according to any one of claims 1 to 4 or 7 to 14, **characterised by** its configuration as an, in particular, implantable medication-dosing device (201) such that the sensor or the first sensor (203) is configured to detect the level of an agent, or a variable dependent thereon, in the body of the patient (P) as a measured value, the evaluating and control device (209, 210) is configured to establish a medication dose per time unit, and the therapy device (212) is configured to administer the established dose to the body per time unit.

## Revendications

1. Appareil de thérapie, en particulier stimulateur cardiaque, appareil de surveillance cardiaque, combinaison stimulateur cardiaque/appareil de surveillance cardiaque (101) ou appareil de dosage de médicament (201), implantable, comportant au moins un détecteur (103 ; 203) pour détecter un paramètre mesurable lors de l'application d'une thérapie prédéterminée dans ou sur le corps d'un patient (P), en particulier caractérisant son état corporel, et pour émettre une valeur de mesure correspondante, un dispositif d'évaluation et de commande (110, 111 ; 209) relié au moins indirectement à la sortie du détecteur et destiné à l'évaluation de la valeur de mesure, y compris son association à l'une parmi au moins deux plages de valeurs prédéterminées, et à la détermination d'un paramètre de commande de thérapie en fonction de ladite évaluation, et un dispositif de thérapie (111.3, 111.4, 112 ; 212) relié à la sortie du dispositif d'évaluation et de commande et destiné à réaliser des thérapies différentes ou des paramètres de thérapie différents en fonction de l'association de la valeur du paramètre de mesure à l'une des plages de valeurs,
**caractérisé en ce que** le dispositif d'évaluation et de commande comprend :
- une mémoire de limite de plage (115 ; 209.3) pour mémoriser au moins deux limites différentes entre deux plages de valeurs du paramètre de mesure et ainsi au moins une zone de superposition entre les plages de valeurs,
- une première unité de comparaison (110.1 ; 209.2) reliée au moins indirectement au détecteur (103 ; 203) via une entrée et à la mémoire de limites de plages (115 ; 209.3) via une autre entrée et destinée à associer la valeur de mesure du paramètre à l'une des plages de valeurs,
- une mémoire de thérapie ( 111.2 ; 211) présentant au moins deux zones de mémorisation adressables séparément pour mémoriser au moins deux valeurs différentes du paramètre de commande de thérapie en association aux valeurs du paramètre de mesure à l'intérieur d'une zone de superposition, chacune conjointement avec un paramètre donné de condition représentant l'historique de paramètre de mesure ou de thérapie, et d'autres zones de mémorisation pour mémoriser une valeur respective du paramètre de commande de thérapie en association à une valeur du paramètre de mesure à l'extérieur d'une zone de superposition,
- une mémoire d'historique (117 ; 209.8) pour mémoriser l'association de plage de la valeur respective précédente du paramètre de mesure ou du paramètre de commande de thérapie d'une thérapie appliquée auparavant à titre de paramètre de condition, et
- des moyens d'évaluation d'historique (118 ; 209.9) pour évaluer la valeur mémorisée avec les valeurs prédéterminées du paramètre de condition et pour émettre à la mémoire de thérapie des données d'adresse exprimant le résultat d'évaluation pour lire exactement une valeur du paramètre de commande de thérapie.

2. Appareil de thérapie selon la revendication 1, **caractérisé en ce qu'**il est prévu une commande de déroulement qui comprend une horloge (106 ; 206), de telle sorte que sous la commande par l'horloge, la valeur actuelle du paramètre de mesure est interrogée périodiquement et comparée avec les limites mémorisées, et un signal de commande d'accès et d'adresse caractérisant le résultat de comparaison est émis, qui provoque ou qui interdit la lecture de la mémoire de thérapie (111.2 ; 211).

3. Appareil de thérapie selon l'une ou l'autre des revendications 1 et 2, **caractérisé par** une réalisation de la commande de déroulement de telle sorte qu'un signal de commande d'accès pour la lecture de la mémoire de thérapie (111.2 ; 211) est émis après constatation du dépassement d'une limite de plage de valeurs.

4. Appareil de thérapie selon l'une des revendications précédentes, **caractérisé en ce qu'**il est prévu une deuxième unité de comparaison (118 ; 209.9) qui est reliée du côté entrée à la première unité de comparaison (110.1 ; 209.2) et à la mémoire d'historique (117 ; 209.8) ainsi que du côté sortie à un sélecteur d'adresse (116 ; 209.4) associé à la mémoire de limite de plage (115) ou à la mémoire de thérapie (111.2 ; 211), pour comparer l'association actuelle de la plage de valeurs du paramètre avec l'association mémorisée.

5. Appareil de thérapie selon l'une des revendications précédentes, **caractérisé par** la réalisation sous la forme d'un stimulateur cardiaque supplétif implantable (111) comportant une mémoire de limite de plage (115) qui est réalisée pour mémoriser au moins une limite entre une plage de taux normal et une plage de taux de bradycardie ainsi que deux limites et ainsi une zone de superposition entre une plage de taux normal et une plage de taux de tachycardie, ainsi qu'une mémoire de thérapie (111.2) qui est réalisée pour mémoriser au moins une thérapie de bradycardie et une thérapie de tachycardie.

6. Appareil de thérapie selon l'une des revendications précédentes, **caractérisé par** la réalisation sous la forme d'un appareil d'anti-tachycardie/anti-fibrillation, en particulier sous la forme d'un stimulateur/défibrillateur d'anti-tachycardie (101), implantable, comportant une mémoire de limite de plage (115) qui est réalisée pour mémoriser au moins une limite entre une plage de taux normal et une plage de taux de tachycardie ainsi que deux limites et ainsi une plage de superposition entre la plage de taux de tachycardie et une plage de fibrillation.

7. Appareil de thérapie selon les revendications 5 et 6, **caractérisé par** la réalisation de la mémoire de limite de plage (115) avec deux zones de mémorisation respectives pour mémoriser deux limites respectives entre la plage de taux normal et la plage de taux de tachycardie ainsi que la plage de taux de tachycardie et la plage de fibrillation.

8. Appareil de thérapie selon l'une ou l'autre des revendications 6 et 7, **caractérisé en ce que** la mémoire de limite de plage (115) comprend des zones de mémorisation pour deux plages de taux de tachycardie adjacentes l'une à l'autre de préférence via une zone de superposition située entre deux limites.

9. Appareil de thérapie selon l'une des revendications précédentes, **caractérisé en ce que** la mémoire de limite de plage (115) comprend deux zones de mémorisation pour mémoriser deux limites et ainsi une zone de superposition entre la plage de taux normal et la plage de taux de bradycardie.

10. Appareil de thérapie selon l'une des revendications précédentes, **caractérisé en ce qu'**une unité de traitement mathématique (107 ; 207) reliée côté entrée à la sortie d'un générateur de valeur de fluctuation (108, 108.1 ; 208, 209.7) est branchée entre la sortie du détecteur (103 ; 203) et l'entrée du dispositif d'évaluation et de commande (110, 111.1, 111.2, 115 ; 209), unité dans laquelle au moins une valeur de fluctuation est imposée à au moins un instant à la valeur de mesure de détecteur sous la commande par une unité de commande de temps (106 ; 206), de telle sorte qu'après évaluation de la valeur de mesure corrigée par la ou par l'une des valeurs de fluctuation, on obtient une valeur du paramètre de commande de thérapie, qui est modifiée par rapport à l'évaluation de la valeur de mesure initiale et/ou de la valeur de mesure corrigée par une autre valeur de fluctuation.

11. Appareil de thérapie selon l'une des revendications précédentes, **caractérisé en ce que** le détecteur (103 ; 203) est réalisé pour détecter un paramètre d'activité ou un paramètre caractérisant une fonction organique du patient (P).

12. Appareil de thérapie selon la revendication 11, **caractérisé par** la réalisation du détecteur ou du premier détecteur sous la forme d'une électrode intracardiaque (203) comportant un amplificateur palpeur (204) agencé en aval pour l'activité électrique du coeur et un dispositif associé pour déterminer la période de l'activité électrique du coeur à titre de valeur de mesure.

13. Appareil de thérapie selon l'une des revendications précédentes, **caractérisé en ce que** le détecteur (103) est réalisé pour détecter le paramètre de thérapie.

14. Appareil de thérapie selon l'une des revendications 10 à 13, **caractérisé en ce que** le générateur de valeur de fluctuation comprend une mémoire de valeur de fluctuation (108) pour plusieurs valeurs de fluctuation et au choix un générateur de nombres aléatoires (108.1) pour choisir l'une des valeurs de fluctuation mémorisées pour l'imposition à la valeur de mesure.

15. Appareil de thérapie selon l'une des revendications 1 à 4 ou 7 à 14, **caractérisé par** la réalisation sous la forme d'un appareil de dosage de médicament (201) en particulier implantable, de telle sorte que le détecteur ou le premier détecteur (203) est conçu pour détecter le niveau d'une substance active ou d'un paramètre qui en dépend dans le corps du patient (P) à titre de valeur de mesure, le dispositif d'évaluation et de commande (209, 210) est conçu pour définir une dose de médicament par unité de temps, et le dispositif de thérapie (212) est conçu pour distribuer au corps la dose définie par unité de temps.
